# EUROPEAN PATENT SPECIFICATION

(11) **EP 1 818 035 B1**
(45) Date of publication and mention of the grant of the patent: **17.07.2013**
(21) Application number: 05809570.4
(22) Date of filing: 24.11.2005
(51) Int. Cl.: A61F 13/49, A61F 13/53, A61F 13/15, A61F 13/472, A61F 5/44

(54) **METHOD FOR PRODUCING AN ABSORBENT SHEET**
VERFAHREN ZUM HERSTELLEN EINES SAUGFÄHIGEN FLÄCHENSTÜCKS
METHODE DE PRODUCTION D'UNE FEUILLE ABSORBANTE

(30) Priority: 30.11.2004 JP 2004347530
(43) Date of publication of application: 15.08.2007
(73) Proprietor: KAO CORPORATION, Tokyo 103-8210 (JP)
(72) Inventor: SATO, Nobuya c/o Kao Corporation, Haga-gun, Tochigi 3213497 (JP); ITOH, Taketo c/o Kao Corporation, Haga-gun, Tochigi 3213497 (JP); MATSUYAMA, Kazuo c/o Kao Corporation, Wakayama-shi, Wakayama 6408404 (JP)
(74) Representative: Vossius & Partner
(86) International application number: PCT/JP2005/021542
(87) International publication number: WO 2006/059524

(56) References cited:
- EP-A1- 1 142 696
- JP-A- 09 067 403
- JP-A- 11 093 073
- JP-A- 2004 149 970
- US-A- 4 537 590
- US-A- 5 962 068
- US-A1- 2003 113 463
- US-A1- 2004 054 342

## Description

This invention relates to a process for producing an absorbent sheet having superabsorbent polymer particles adhering to constituent fibers thereof.

Various types of absorbent sheets including a fibrous base and superabsorbent polymer particles adhered to the constituent fibers of the fibrous base are known (see, for example, JP 9-67403A and JP 11-93073A). Such absorbent sheets are suited for use as an absorbent member of absorbent articles such as disposable diapers and sanitary napkins or a water-retaining material in the fields of agriculture and civil engineering.
US 2003/0113463 discloses a process for forming an absorbent fibrous web composite.
US 5 962 068 discloses a water-absorptive composite comprising a fibrous substrate bearing water-absorptive polymer particles.
EP 1 142 696 discloses a water-absorbing composite comprising water-absorbing polymer particles.
US 4 537 590 discloses a superthin absorbent disposable product.
US 2004/0054342 discloses absorbent articles including an absorbent material that contains superabsorbent polymer non-adhesively attached to and restrained by a nonwoven.
Being thin and highly flexible, the absorbent sheet used an absorbent member provides an absorbent article giving comfort and improved fit to the body of a wearer while worn.

Nevertheless, a disposable diaper or a sanitary napkin often moves out of position as a wearer actively moves, which can cause leakage. Therefore, an absorbent article providing a better fit has been demanded.

The present invention relates to a process for producing an absorbent sheet having stretchability and having a fibrous base material which contains fibers and superabsorbent polymer particles bonded to the fibers. The superabsorbent polymer particles are the result of applying a liquid which contains a polymerizable monomer and/or an oligomer thereof to the fibers and polymerizing the monomer and/or the oligomer.
The present invention provides a process of producing an absorbent sheet as claimed in any of claims 1 to 4. The process includes the steps of transversely aligning continuous filaments as fibers constituting a fibrous base material, applying a liquid containing a polymerizable monomer and/or an oligomer thereof to the continuous filaments, and causing the monomer and/or the oligomer to polymerize into superabsorbent polymer particles bonded to the continuous filaments.

Fig. 1 schematically shows the structure of an absorbent article having an absorbent sheet (not part of the invention).
Fig. 2 schematically shows the structure of another absorbent article having an absorbent sheet (corresponding to Fig. 1).
Fig. 3 schematically shows the structure of still another absorbent article having an absorbent sheet (corresponding to Fig. 1).
Fig. 4 schematically illustrates a process according to the present invention of making an absorbent sheet

The absorbent sheet has a stretchable fibrous base and superabsorbent polymer particles adhering to the fibers constituting the fibrous base. The superabsorbent polymer particles are the result of adhering a liquid containing a polymerizable monomer and/or an oligomer thereof to the fibers and causing the monomer and/or the oligomer to polymerize. That is, the superabsorbent polymer particles are the result of *in situ* polymerization on the fibers constituting the fibrous base. The absorbent sheet is characterized by the superabsorbent polymer obtained by the *in situ* polymerization of the present invention on the base and by its stretchability

The fibrous base can be formed of various fibrous sheets, including nonwovens, wovens, knits, opened tows, tow-opening webs, and pads. These fibrous sheets have stretchability or latent stretchability. The term "latent stretchability (or latently stretchable)" as used herein means the property of a sheet of not having stretchability as such but developing stretchability upon being subjected to a prescribed treatment. Examples of the fibrous sheets having stretchability include those containing crimped fibers, elastic resin fibers or mixtures thereof as constituent fibers. Examples of the fibrous sheets containing elastic resin fibers include spun-bonded or melt-blown nonwoven fabrics made out of urethane, styrene or olefin elastomers.

Examples of the fibrous sheets having latent stretchability include those containing self-crimping fibers as constituent fibers. A fibrous sheet containing self-crimping fibers creates crimps of the fibers on being heated to a predetermined temperature and thereby becomes stretchable. Examples of self-crimping fibers include polyester/polyolefin bicomponent conjugate fibers having a sheath/core or side-by-side configuration.

It is preferred to use, as a fibrous base, a stretchable or latently stretchable nonwoven fabric or a stretchable or laterally stretchable sheet composed of continuous filaments, such as an opened tow, in view of ease and cost of the production of the absorbent sheet. *Inter alia,* using an opened tow has the advantage of providing an absorbent member for an absorbent article having a reduced thickness and a reduced weight per unit area with the absorption capacity being equal to that of conventional absorbent members. There is offered another advantage that the structure of the absorbent member is less likely to be destroyed by vigorous movement of a wearer.

The fiber constituting the fibrous base is not particularly limited in material, and various synthetic, semisynthetic and natural fibers can be used. Examples of suitable synthetic fibers are polyester fiber, polyethylene fiber, polypropylene fiber, polyvinyl alcohol fiber, acrylic fiber, and polyamide fiber. Examples of suitable semisynthetic or natural fibers are acetates, rayon, and cotton. The constituent fiber may be a mixture of two or more of the recited fibers. These fibers may be subjected to a hydrophilizing treatment or a hydrophobilizing treatment, which depends on the intended use of the absorbent sheet. These fibers are used in the form of either staple fiber or continuous fiber, which depends on the process adopted to make the fibrous base. For example, staple fiber is used where the fibrous base is a carded web or an air-through or hydroentangled nonwoven fabric prepared from a carded web. Continuous fibers (continuous filaments) are used where the fibrous base is an opened tow or a spun-bonded nonwoven fabric. When in using fiber that absorbs liquid to swell or is porous and takes in liquid, the viscosity of the liquid containing a polymerizable monomer and/or an oligomer thereof should be adjusted relatively high so as not to be confined inside the fibers. It is possible to take good advantage of the increase in viscosity of the liquid with the progress of polymerization. In order to permit use of various kinds of liquids, it is preferred to use fibers which hardly absorb liquid and have a surface tension such that liquid applied thereto takes on a stable form.

The fiber constituting the fibrous base is not particularly limited in thickness, either. A thickness is selected as appropriate to the intended use of the absorbent sheet. For use as an absorbent member of an absorbent article, for instance, fibers having a fineness of 1 to 40 dtex are usually used, and fibers having a fineness of 1 to 30 dtex are preferably used. When, in particular, a fibrous base having a relatively large interfiber distance is used to secure absorption and retention of a highly viscous liquid, the fineness of the constituent fibers is preferably 5 to 40 dtex, more preferably 10 to 30 dtex. In designing a thin and light absorbent sheet, the fineness of the constituent fibers is preferably 1 to 5 dtex, more preferably 1 to 3 dtex.

In the cases where the fiber constituting the fibrous base is crimped fiber, the crimped fiber preferably has a percentage of crimp (JIS L0208) of 20% to 90%, more preferably 40% to 90%, even more preferably 50% to 80%. Using crimped fiber having a crimp percentage within the recited range provides the fibrous base with sufficiently high stretchability and allows for stable adhesion of a large quantity of superabsorbent polymer particles to the fibrous base. The means for crimping fibers is not particularly limited. The crimp may be either two-dimensional or three-dimensional. The percentage of crimp is defined to be a percentage of a difference between the length A of a crimped fiber in its straightened state and the natural length B of the crimped fiber to the length A.

The number of crimps of the crimped fibers desirably having the recited percentage of crimp is preferably 2 to 25, more preferably 4 to 20, even more preferably 10 to 20, per centimeter.

The weight per unit area of the fibrous base is decided as appropriate for the intended use of the absorbent sheet. For use as an absorbent member of an absorbent article, for instance, the fibrous base preferably weighs 20 to 100 g/m², more preferably 30 to 60 g/m² to provide a thin and flexible absorbent member.

The superabsorbent polymer particles adhering to the constituent fibers of the fibrous base are not particularly limited in material as long as they are formed as a result of polymerization of a polymerizable monomer and exhibit water absorbency. It is preferred that the superabsorbent polymer be capable of absorbing 20 times or more their own weight in physiological saline. Typical examples of such a superabsorbent polymer include, but are not limited to, polyacrylic acid salts and methacrylic acid salts.

The amount of the superabsorbent polymer adhering to the fibrous base is chosen as appropriate to the intended use of the absorbent sheet. For use as an absorbent member of an absorbent article, for example, the amount is preferably 50 to 500 g/m², more preferably 100 to 350 g/m², to provide a thin and flexible absorbent member with sufficient absorption capacity.

The superabsorbent polymer particles preferably have a mean particle size of 50 to 1000 µm, more preferably 100 to 600 µm, to secure improved absorbency of the absorbent sheet. The particle size is measured with magnification under an optical microscope. Two hundred particles are measured to obtain a mean particle size (median diameter).

The superabsorbent polymer particles may adhere to the constituent fibers of the fibrous base like beads on a string or in the form of agglomerates of plural particles.

A ratio of the basis weight of the fibrous base to the basis weight of the superabsorbent polymer (fibrous base/superabsorbent polymer) in the absorbent sheet is preferably 1/1 to 1/10, more preferably 1/2 to 1/6, to provide a thin and flexible absorbent member with sufficient absorbing capacity.

The weight of the absorbent sheet is preferably 30 to 500 g/m², more preferably 50 to 300 g/m², to provide a thin and flexible absorbent member while securing sufficient absorbing capacity. The thickness of the absorbent sheet is preferably 0.1 to 10 mm, more preferably 0.2 to 1 mm, to the same effect.

The absorbent sheet which is stretchable may have the stretchability in the machine direction (MD) or the cross direction (CD) or in both the MD and CD. Whichever the direction may be, the absorbent sheet is stretchable in at least one direction, and the stretch in the direction is such that the stress at 20% elongation ranges preferably 0.2 to 4.0 N, more preferably 0.5 to 3.0 N, per centimeter width, to exhibit sufficient stretchability. Such stretchability is particularly preferred when the absorbent sheet is used as an absorbent member of an absorbent article so that the absorbent member may follow the wearer's movement to maintain a good fit. The stress measurement is made with a tensile tester under conditions of an initial gauge length of 50 mm, a specimen width of 25 mm, and a pulling speed of 300 m/min.

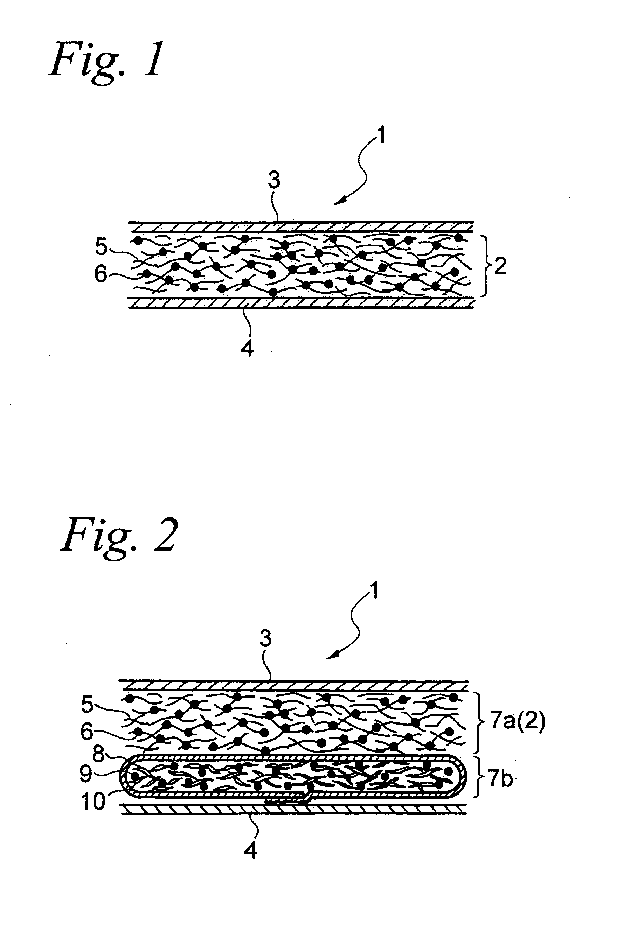

An example of an absorbent article using the absorbent sheet produced with the process according to the present invention as an absorbent member is illustrated in Fig. 1. Fig. 1 presents a cross-section of the absorbent article along the thickness direction. The absorbent article 1 shown has an absorbent sheet 2. A liquid permeable topsheet 3 is disposed on one side of the absorbent sheet 2, and a liquid impermeable or repellent (hereinafter inclusively referred to as "liquid impermeable") backsheet 4 is disposed on the other side. The absorbent sheet 2 has a fibrous base 5 and superabsorbent polymer particles 6 adhering to the constituent fibers of the fibrous base 5. In the absorbent sheet 2 shown in Fig. 1, the fibrous base 5 is nonwoven fabric of staple fiber to which the superabsorbent polymer particles 6 adhere.

According to the structure of the absorbent article 1 illustrated in Fig. 1, because the absorbent sheet 2 is stretchable, the absorbent article 1 is capable of maintaining a snug fit while worn by a wearer even when the wearer moves vigorously. In this application, it is preferred for the absorbent sheet 2 to be stretchable in at least one of the longitudinal and lateral directions of the absorbent article 1. It is more preferred for the absorbent sheet 2 to be stretchable in both the longitudinal and lateral directions. According to the structure of Fig. 1, since the superabsorbent polymer particles 6 firmly adhere to the fibrous base 5, they hardly fall off. This means that there is no more need to use tissue that has been used to wrap an airlaid structure of fluff pulp and superabsorbent polymer particles in conventional absorbent articles to prevent fall-off of the particles. Therefore, the absorbent article 1 can be formed out of a reduced number of elements, leading to simplification of production steps and reduction of production cost.

In the case where a latently stretchable absorbent sheet is used to assemble an absorbent article, the absorbent sheet is subjected to a prescribed treatment before assembly. When, for example, the latently stretchable absorbent sheet contains self-crimping fiber, heat at a predetermined temperature is applied to the sheet to crimp the self-crimping fiber thereby making the sheet develop stretchability. The resulting absorbent sheet having developed stretchability is used as an absorbent member in the assembly.

To further improve the fit of the absorbent article 1 having the stretchable absorbent sheet 2, it is preferred that the topsheet 3 and the backsheet 4 as well as the absorbent sheet 2 be stretchable in the same direction as the absorbent sheet 2.

Various types of stretchable topsheets have been proposed in the field of absorbent articles. A typical example for use in the invention is the sheet disclosed in commonly assigned JP 2002-187228A. The sheet disclosed comprises a nonwoven fabric having an upper layer and a lower layer partly joined together in the thickness direction. The lower layer contains heat shrinkable fibers having shrunken, and the upper layer contains fibers having substantially no heat shrinkability. When the heat shrinkable fibers in the lower layer shrink, in-plane shrinkage of the lower layer occurs. The shrinking force raises the upper layer between the joints to create an uneven surface on the upper layer. Using self-crimping fiber as the heat shrinkable fiber makes the sheet stretchable.

Various types of stretchable backsheets have also been proposed in the field of absorbent articles. For example, film of ester or urethane elastomers can be used as the backsheet 4. A stretchable, nonporous, moisture permeable sheet is also used preferably to help control humidity inside the article while worn. Materials of such a sheet include thermoplastic urethane elastomers composed of a block having a urethane bond as a hard segment and a block having polycarbonate polyol, ether polyol, caprolactone polyester or adipate polyester as a soft segment and thermoplastic ester elastomers composed of a block having aromatic polyester as a hard segment and a block having aliphatic polyether or aliphatic polyester as a soft segment. The nonporous sheets of these materials owe the moisture permeability not to fine pores but to the elastomer's properties of taking in, diffusing, and releasing water vapor. Where moisture permeability is not demanded, a sheet made out of an ethylene-α-olefin thermoplastic elastomer having a density of 0.90 g/cm³ or less, preferably 0.87 g/cm² or less, prepared using a metallocene catalyst is suitable. Examples of suitable α-olefins are propylene, butene, hexene, and octene. These sheets preferably have a weight of 15 to 50 g/m² and a thickness of 10 to 100 µm.

An absorbent article is generally composed of three fundamental elements - the topsheet, the backsheet, and the absorbent member, to which various optional elements are added according to necessity in view of improving various properties of the absorbent article. For example, a pair of leg flaps extending laterally outward from both long sides of an absorbent article or a pair of gathered cuffs standing from near both long side edges of an absorbent member and extending along the longitudinal direction of an absorbent article may be used as main elements other than the three where necessary. When the absorbent article 1 shown in Fig. 1 is provided with additional main elements other than the three, such as leg flaps and gathered cuffs, it is preferred that such additional elements be stretchable, too. Leg flaps or gathered cuffs of common absorbent articles are in most cases stretchable because they are usually elasticized with an elastic strand, etc. fixed thereto in its extended state.

In cases where all the main elements composing an absorbent article are stretchable and assembled with their stretchability being in the same direction, the resulting absorbent article has stretchability as a whole. Even when a wearer wearing the stretchable absorbent article vigorously moves his or her body, the absorbent article extends and contracts in conformity with the movement. Thus, the stretchable absorbent article is prevented from sliding out of position more effectively, making leakage ascribed to sliding much less likely to occur.

Modifications of the absorbent article of Fig. 1 include an absorbent article 1 illustrated in Fig. 2. The absorbent article 1 of Fig. 2 has an absorbent member of dual layer structure composed of an upper absorbent layer 7a and a lower absorbent layer 7b. The upper absorbent layer 7a, being the same as in the absorbent article in Fig. 1, is an absorbent sheet having a fibrous base 5 and superabsorbent polymer particles 6 adhering the constituent fibers of the fibrous base 5. The lower absorbent layer 7b is a structure having an airlaid body of fluff pulp 8 and superabsorbent polymer particles 9 wrapped in absorbent paper such as tissue. The lower absorbent layer 7b has practically the same structure as an absorbent member used in conventional absorbent articles. The absorbent article 1 in Fig. 2 has a higher absorption capacity than that of Fig. 1. Accordingly, the absorbent article 1 of Fig. 2 is especially suited for use intended to absorb and retain large quantities of liquid, for example, as a disposable diaper.

In the article shown in Fig. 2, the fluff pulp 8 in the lower absorbent layer 7b preferably has a weight of 30 to 300 g/m², more preferably 50 to 200 g/m², to secure sufficient absorption capacity. The superabsorbent polymer 9 preferably weighs 30 to 300 g/m², more preferably 50 to 200 g/m², for the same reason.

A ratio of the basis weight of the upper absorbent layer 7a to the basis weight of the lower absorbent layer 7 (upper/lower) is preferably 1/5 to 3/l, more preferably 1/3 to 2/l, to secure sufficient absorption capacity while enjoying the advantages of using the absorbent sheet 2, i.e., thinness and flexibility.

It is desirable for the lower absorbent layer 7b to be stretchable. A stretchable lower absorbent layer 7b can be obtained by using, for example, fluff pulp, helically crimped synthetic resin fibers, and superabsorbent polymer particles. In such a stretchable lower absorbent layer, the individual helically crimped fibers entangle at least one, preferably two or more, fluff pulp fiber(s), and at least one, preferably two or more, superabsorbent polymer particle(s) in the helical crimp (coil). Seen microscopically, the lower absorbent layer 7b has a countless number of fiber composites each composed of the helically crimped fiber, fluff pulp fiber and/or superabsorbent polymer particle. The fiber composites are distributed in the absorbent layer practically uniformly. As a whole, the absorbent layer exhibits stretchability owing to the stretchability of the helically crimped fibers.

The helically crimped fiber is the result of heating to cause self-crimping fiber to shrink in a helical coil. It is preferred that the helically crimped fiber shrink to develop a helical crimp while engulfing fluff pulp and/or superabsorbent polymer particles in the helical crimp to form a fiber composite. Since self-crimping fiber (fiber before crimp development) can be processed in the same manner as for general fiber, it can be uniformly distributed in an absorbent layer in the same manner as in the manufacture of a general absorbent member, for example, by an air-laying method. Heating following the uniform distribution causes the self-crimping fibers to develop crimps, resulting in a great number of the fiber composites containing the crimped fibers uniformly distributed throughout the absorbent layer. In this way, stretchability ascribed to the helically crimped fibers is fully manifested. A stretchable absorbent member having such a structure is described, e.g., in commonly assigned JP 2004-159786.

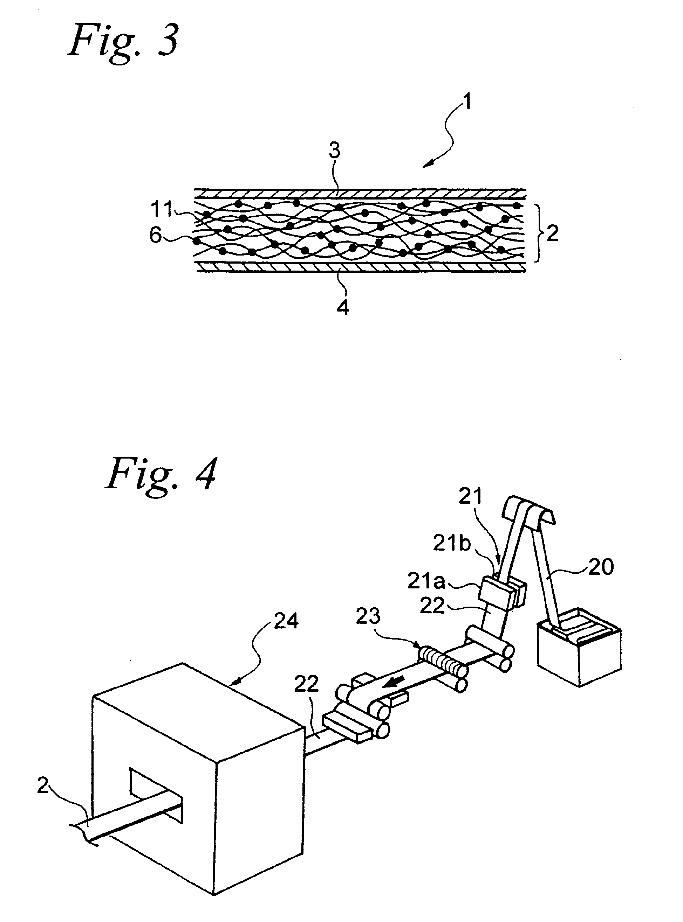

Another absorbent article 1 is illustrated in Fig. 3. This absorbent article 1 represents an example of using, as an absorbent member, an absorbent sheet 2 having an opened tow 11 and superabsorbent polymer particles 6 adhering to continuous filaments constituting the opened tow 11. According to this configuration, the absorbent sheet 2 is thinner and lighter and is less likely to be destroyed structurally even by the active movement of a wearer.

The continuous filaments of the opened tow 11 preferably have hydrophilic properties. Hydrophilic continuous filaments that can be used in the present embodiment include those essentially having hydrophilicity and those essentially having no hydrophilicity but endowed with hydrophilicity by a hydrophilizing treatment. Preferred examples of the latter are continuous filaments of polypropylene or polyester having been rendered hydrophilic by a hydrophilizing treatment. Preferred examples of the former are continuous filaments of cellulose acetate, nylon or rayon. Cellulose acetate continuous filaments are particularly preferred; for they maintain bulkiness even on wetting.

The continuous filaments of the opened tow 11 are oriented along one planar direction of the absorbent article 1. With the continuous filaments oriented unidirectionally, a body fluid absorbed into the absorbent article 1 diffuses preferentially in the orientation direction of the continuous filaments, namely, in the planar direction of the absorbent article. Conversely, diffusion of the liquid in a direction perpendicular to the orientation direction is suppressed. When the continuous filaments are in a longitudinal alignment with the absorbent article 1, leakage from the lateral sides of the absorbent article 1 (side leakage) is effectively prevented.

The continuous filaments may be oriented in the transverse direction of the absorbent article 1, in which case liquid diffusion in the longitudinal direction of the article 1 is suppressed, and downward wicking properties are obtained. In this case, it is preferred for the opened tow 11 to have linear adhesion joints across the orientation direction of the continuous filaments in order to prevent leakage from lateral sides (side leakage). The term "linear" as used herein means a continuous line for suppressing liquid wicking. Individual joints such as linear seals do not have to be connected to each other. For example, when linear seals are discretely aligned to overlap with each other in a direction to block liquid migration, a group of the linear seals can be said to be linear. The line may be straight, curved, or kinked. The line width is preferably about 0.2 to 15 mm.

The absorbent sheet 2 used in the absorbent article 1 is preferably produced as follows. As illustrated in Fig. 4, a tow band 20 is pulled out and fed to a tow opening unit 21 where it is spread to a prescribed width to become an opened tow 22. Any tow opening device commonly in the art is appropriately used as the unit 21 with no particular restriction. The tow opening unit used in this particular embodiment is of the type in which an air banding jet is used. This unit includes an air blowing member 21a and an air suction member 21b. The blowing member 21a and the suction member 21b are placed to face each other between which the tow 20 runs. The blowing member 21a has a large number of nozzles (not shown) arrayed along the width direction of the tow 20, from which an air stream is jetted at a predetermined pressure across the whole width of the tow 20. The tow 20 is thus opened or spread to a prescribed width by the air pressure. The blown-out air is recovered by the suction member 21b.

The opened tow 22 is then passed on a tow-opening roll 23, where the tow is further spread and stabilized in the opened state. The roll 23 is a grooved roll having a large number of parallel grooves threaded along the direction of rotation. Such a tow-opening roll is well known in the art.

The opened tow 22 is transported to a reaction chamber 24. While being transported, the opened tow 22 is stretched from its shrinking state under tension caused by transportation. The opened tow 22 is thus introduced into the reaction chamber 24 in its stretched state. In the reaction chamber 24, a liquid containing a polymerizable monomer and/or an oligomer thereof is applied to the opened tow 22, and the polymerizable monomer and/or the oligomer thereof is/are caused to polymerize, resulting in the formation of superabsorbent polymer particles adhering to the fibers of the tow. The polymerizable monomer is not particularly limited. Any polymerizable monomer capable of polymerizing to produce an absorbent polymer can be used. Such a type of monomers are well known in the art. Typical examples are found in JP 9-67403A and JP 11-93073A cited *supra.*

The liquid is preferably applied to the opened tow 22 in the form of droplets so that the polymerization may proceed stably, and the resultant polymer may be fixed more uniformly and less likely to come off the fibers. Conversion of the liquid into droplets can be achieved by known liquid pulverization techniques, such as a dropping method, an atomizing method using a spray nozzle, an atomizing method using a rotary disc atomizer, and an ultrasonic method. The size of droplets is preferably 10 to 1000 µm, more preferably 50 to 800 µm.

Polymerization of the polymerizable monomer and/or the oligomer in the liquid adhered to the opened tow 22 can be effected by, for example, a method using a polymerization initiator or a method of irradiating with a radiation, an electron beam or ultraviolet rays. Examples of polymerization initiators in the case of using an addition polymerizable monomer, namely addition polymerization initiators include radical generating initiators, such as peroxides (e.g., hydrogen peroxide, benzoyl peroxide, and cumene hydroperoxide), azo compounds (e.g., azobisisobutyronitrile), ammonium persulfate, and potassium persulfate; and redox initiator systems, such as combinations of these radical generators and reducing agents, e.g., sodium hydrogensulfite, L-ascorbic acid, and iron (II) salts. In the case of using a polyaddition polymerizable monomer, tertiary amines or tin compounds are suitably used as a catalyst for polyaddition reaction.

In the case of using a redox initiator system as a polymerization initiator, the following manner of polymerization is preferred. Droplets of a polymerizable monomer A containing an oxidizing agent, one of the components of a redox initiator system, and droplets of a polymerizable monomer B containing a reducing agent, the other component of a redox initiator system, are mixed in a gaseous phase to form an oligomer. The droplets of the oligomer are adhered to the opened tow 22. The manner of polymerization described is graphically shown in Fig. 1 of JP 9-67403A. In another preferred manner, droplets of the polymerizable monomer A and droplets of the polymerizable monomer B are simultaneously or successively applied to the opened tow 22, brought into contact with each other on the opened tow 22, and caused to redox-polymerize there. Compared with the manner in which the droplets of the polymerizable monomer A and the droplets of the polymerizable monomer B are mixed in a gaseous phase, the latter manner of polymerization attains a higher throughput to achieve increased productivity of the absorbent sheet 2. The latter manner of polymerization is described, e.g., in commonly assigned JP 2003-134382A. The polymerization method by irradiation with ultraviolet rays is described, e.g., in commonly assigned JP 2004-52117A.

A known crosslinking agent may be added to a polymerization system to obtain a superabsorbent polymer having desired absorbency. With respect to the details of the polymerization reaction in the reaction chamber 24, reference can be made to JP 9-67403A and JP 11-93073A cited *supra.*

The opened tow 22 is then subjected to appropriate post treatment steps, such as extraction of unreacted monomers and dehydration/drying. The opened tow 22 may also be subjected to the step of shaping into a narrower band primarily for the purpose of equalize the distribution of superabsorbent polymer particles in the thickness direction of a finally obtained absorbent sheet 2. The step of narrowing is performed by gathering the continuous filaments, piling the opened tows having been narrowed by gathering the continuous filaments, folding the opened tow, or a combination of these operations. There is thus obtained an absorbent sheet 2 having the superabsorbent polymer particles adhering to the continuous filaments of the opened tow.

According to this process, superabsorbent polymers can be adhered to crimped continuous filaments while the filaments are in their stretched state under tension. After completion of the polymerization reaction, the filaments are released from tension. Thereupon the superabsorbent polymers are held in numerous spaces formed by crimped continuous filaments, and moreover the superabsorbent polymer particles can be embedded deep into the sheet. As a result, the superabsorbent polymer particles, even when present in a large amount, are still less likely to move extremely in the sheet or fall off the sheet. Additionally, even when a wearer vigorously moves his or her body, the structure of the absorbent sheet 2 is still less likely to be destroyed. Furthermore, because the superabsorbent polymer particles can be three-dimensionally distributed in the sheet, gel blocking is prevented effectively.

The process of producing an absorbent sheet illustrated in Fig. 4 is for the case in which a stretchable opened tow is used as a fibrous base. The same process also applies to the production of an absorbent sheet in which a fibrous base other than an opened tow is used. The absorbent sheet can also be produced by processes other than the one illustrated in Fig. 4, for example, the processes shown in Figs. 1 and 2 of JP 11-93073A cited *supra.* That is, fibers are joined together into a fibrous base in the step of polymerizing a liquid containing a polymerizable monomer and/or an oligomer thereof.

While a stretchable fibrous base is used in the process illustrated in Fig. 4, the process is applicable to an embodiment in which a fibrous base having no stretchability is used, and a superabsorbent polymer is produced *in situ* on fibers constituting the non-stretchable fibrous base. For instance, a tow of non-stretchable continuous filaments is opened, and a superabsorbent polymer is produced on the continuous filaments of the opened tow. This process and the process of Fig. 4 are characterized in common in that superabsorbent polymer particles are formed while adhering to continuous filaments widely aligned or spread in the transverse direction so that the particles may adhere over a broad area to attain an increased adhesion efficiency. After the formation of polymer particles, the widely spread continuous filaments may be shaped into a band with a smaller width in various manners so as to design the distribution of the particles as appropriate, which is another characteristic of both processes. For example, the tow band may be opened to a slightly smaller width, and the opened tow having superabsorbent polymer particles adhering thereto is folded with the particles-adhered side inward to provide an absorbent sheet having a larger amount of the superabsorbent polymer in the inside. Conversely, the opened tow may be folded with the particles-adhered side outward to provide an absorbent sheet having a larger amount of the superabsorbent polymer in the outer side thereof. On the other hand, the tow band may be opened to a larger width, and the opened tow having superabsorbent polymer particles adhered thereto may be folded to give an absorbent sheet having the superabsorbent polymer uniformly distributed throughout the sheet. These manipulations can be selected as appropriate to the purpose of use. These processes for adhering superabsorbent polymer to continuous filaments allows for adhesion of small-sized superabsorbent polymer particles while suppressing formation of large-sized particles due to agglomeration even when the continuous filaments are thin having a fineness as small as about 1 to 3 dtex. In both the process using non-stretchable fibrous base and the process illustrated in Fig. 4, the superabsorbent polymer particles adhere to continuous filaments transversely aligned to wide width. When the fibers are crimped fibers, therefore, the process of Fig. 4 is followed, in which satisfactory fiber alignment can be established by producing the polymer particles while adhering to the continuous filaments held in the stretched state by longitudinal tension. Accordingly, when a stretchable absorbent sheet is desired, crimped continuous filaments are used in accordance with the process of Fig. 4. Conversely, when stretchability is unnecessary, an absorbent sheet is produced using non-crimped continuous filaments in accordance with the other process.

As described in detail, the absorbent sheet produced with the process of the present invention is particularly suited for use as an absorbent member of various absorbent articles. Examples of the absorbent articles to which the absorbent sheet finds application include disposable diapers, sanitary napkins, and incontinence pads. Applications to which the absorbent sheet is particularly suited are pants type absorbent articles, including disposable pull-on diapers and pants type sanitary napkins, in view of fit to the body of a wearer and conformability to the wearer's motion.

The absorbent sheet also finds suitable applications to a soil water-retaining material for agricultural or civil engineering use as well as an absorbent member of absorbent articles.

In another method alternative to the process shown in Fig. 4, a fibrous base that develops stretchability on being heated is used. In this case, after a liquid containing a polymerizable monomer and/or an oligomer thereof is applied to the fibrous base, followed by polymerizing the monomer and/or oligomer, the fibrous base can be heated to develop stretchability. In the case when heat is used to carry out polymerization, polymerization of the monomer/oligomer and development of stretchability of the fibrous base can be effected simultaneously. In either manner, transportation of the fibrous base is preferably done while controlling the degree of stretchability development in the CD of the fibrous base by means of, for example, a pin tenter. Alternatively, the degree of stretchability development of the fibrous base is preferably controlled during transportation by lowering the outlet speed than the inlet speed.

As described, the absorbent sheet obtained with the process of the present invention has stretchability. When used as, for example, an absorbent member of an absorbent article, it extends and contracts in conformity with the body of a wearer even when the wearer vigorously moves his or her body. The absorbent article is thus prevented from sliding out of position, hardly causing leakage. Since the superabsorbent polymer particles firmly adhere to the fibers, the polymer particles hardly fall off the fibers even when adhering in a large amount. Even when the wearer vigorously moves, the superabsorbent polymer particles are less likely to be moved and localized, and the absorbent member itself is less likely to be destroyed structurally.

## Claims

1. A process for producing an absorbent sheet comprising the steps of:
applying a liquid containing a polymerizable monomer and/or an oligomer thereof to a fibrous base material having stretchability, in a stretched state thereof, and
causing the monomer and/or the oligomer to polymerize into superabsorbent polymer particles bonded to fibers of the fibrous base material,
wherein the fibrous base material comprises
a stretchable nonwoven fabric or stretchable sheet composed of continous filaments.

2. A process for producing an absorbent sheet according to claim 1 further comprising the steps of:
transversely aligning continuous filaments as fibers constituting a fibrous base material,

3. The process according to claim 2, further comprising the step of narrowing the width of the fibrous base material having the superabsorbent polymer particles bonded thereto.

4. The process according to claim 2, further comprising the step of narrowing the width of the fibrous base material comprising the continuous filaments by any one of means of gathering, piling, folding, and a combination thereof.

## Patentansprüche

1. Ein Verfahren zur Herstellung einer saugfähigen Bahn, umfassend die Schritte:
Aufbringen einer Flüssigkeit, enthaltend ein polymerisierbares Monomer und/oder ein Oligomer davon, auf ein dehnbares, in einem gedehnten Zustand befindliches Fasergrundmaterial, und
Veranlassen des Monomers und/oder des Oligomers zu superabsorbierenden, an Fasern des Fasergrundmaterials gebundenen Polymerpartikeln zu polymerisieren, wobei das Fasergrundmaterial einen dehnbaren Vliesstoff oder eine dehnbare Bahn,
zusammengesetzt aus kontinuierlichen Filamenten, umfasst.

2. Ein Verfahren zur Herstellung einer saugfähigen Bahn nach Anspruch 1, weiter umfassend die Schritte:
transversales Anordnen von kontinuierlichen Filamenten, wobei die Fasern ein Fasergrundmaterial bilden.

3. Das Verfahren nach Anspruch 2, weiter umfassend den Schritt der Verringerung der Breite des Fasergrundmaterials mit den daran gebundenen superabsorbierenden Polymerpartikeln.

4. Das Verfahren nach Anspruch 2, weiter umfassend den Schritt der Verringerung der Breite des Fasergrundmaterials, umfassend die kontinuierlichen Filamente, durch eine der Maßnahmen Raffen, Stapeln, Falten und eine Kombination davon.

## Revendications

1. Procédé pour fabriquer une feuille absorbante comprenant les étapes de :
l'application d'un liquide contenant un monomère polymérisable et/ou un oligomère de celui-ci sur un matériau de base fibreux possédant une aptitude à l'étirage, dans un état étiré de celui-ci, et
l'entraînement de la polymérisation du monomère et/ou de l'oligomère en particules polymères super-absorbantes liées à des fibres du matériau de base fibreux,
dans lequel le matériau de base fibreux comprend un tissu non tissé étirable ou une feuille étirable composée de filaments continus.

2. Procédé pour fabriquer une feuille absorbante selon la revendication 1, comprenant en outre les étapes de :
l'alignement transversal de filaments continus en tant que fibres constituant un matériau de base fibreux.

3. Procédé selon la revendication 2, comprenant en outre l'étape du rétrécissement de la largeur du matériau de base fibreux possédant les particules polymères super-absorbantes liées à celui-ci.

4. Procédé selon la revendication 2, comprenant en outre l'étape du rétrécissement de la largeur du matériau de base fibreux comprenant les filaments continus grâce à un quelconque des moyens de rassemblement, d'empilage, de pliage, et d'une association de ceux-ci.
